(19) Europäisches Patentamt
European Patent Office
Office européen des brevets

(11) Publication number: 0 151 449
B1

(12) **EUROPEAN PATENT SPECIFICATION**

(45) Date of publication of patent specification: 20.04.88

(51) Int. Cl.$^4$: C 07 C 102/00, C 07 C 103/375, C 07 D 263/10

(21) Application number: 85100836.7

(22) Date of filing: 28.01.85

(54) **Process for the production of fluorine containing N-(beta-bromoethyl)amide and of 2-(perfluoroalkyl)-1,3-oxazoline.**

(30) Priority: 31.01.84 JP 14483/84

(43) Date of publication of application: 14.08.85 Bulletin 85/33

(45) Publication of the grant of the patent: 20.04.88 Bulletin 88/16

(84) Designated Contracting States: DE FR GB

(56) References cited:
US-A-3 293 245
US-A-3 564 004

JOURNAL OF ORGANIC CHEMISTRY, vol. 30, no. 11, November 1965, pages 3729-3733, Washington, D.C., US;H. BROWN et al.: "Reactions of perfluoroalkyl nitriles. VI. Perfluoroacyl imidates as intermediates"

(73) Proprietor: ASAHI GLASS COMPANY LTD.
No. 1-2, Marunouchi 2-chome
Chiyoda-ku, Tokyo (JP)

(72) Inventor: Ishikawa, Nobuo
10-10, Shinoharadai-machi Kohoku-ku
Yokohama-shi Kanagawa-ken (JP)
Inventor: Sekiya, Akira
No. 5791, Hino-cho Konan-ku
Yokohama-shi Kanagawa-ken (JP)

(74) Representative: Wächtershäuser, Günter, Dr.
Tal 29
D-8000 München 2 (DE)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel process for producing an N-(β-bromoethyl)amide of a fluorine-containing carboxylic acid and/or 2-(perfluoroalkyl)-1,3-oxazoline.

For the production of 2-substituted-1,3-oxazoline derivatives, the following methods have been known.

a) A method wherein β-acetamido ethanol is heated to a temperature of from 260 to 280°C, or β-bromoethylamine hydrobromide is boiled together with acetic anhydride to obtain 2-methyl-1,3-oxazoline.

b) A method wherein β-bromopropylamine hydrobromide is boiled together with acetic anhydride to obtain 2,5-dimethyl-1,3-oxazoline.

c) A method wherein N-(β-bromoethyl)benzamide is treated with an alkali, or an ether solution of benzimino(β-chloroethyl)ether is evaporated over sulfuric acid desiccator, or N-benzoylethyleneimine was distilled to obtain 2-phenyl-1,3-oxazoline.

d) A method wherein 2-trifluoromethyl-1,3-oxazoline is obtained from trifluoroacetonitrile and 2-chloroethyl alcohol.

Among these methods for the production of 2-substituted-1,3-oxazoline derivatives, a method for obtaining 2-(perfluoroalkyl)-1,3-oxazoline is quite rare, and an effective method has not yet been found.

The present inventors have conducted extensive researches to develop a process for advantageously producing a 2-(perfluoroalkyl)-1,3-oxazoline represented by the formula

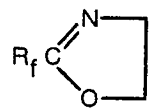

As a result, they have found a method for producing the compound and/or an N-(β-bromoethyl)amide of a fluorine-containing carboxylic acid in an extremely good yield. Namely, present invention provides a process for producing an N-(β-bromoethyl)amide of a fluorine-containing carboxylic acid represented by the formula:

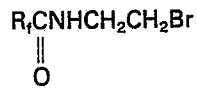

(II)

wherein $R_f$ is a perfluoroalkyl group having from 1 to 20 carbon atoms, and/or a fluorine-containing 2-oxazoline represented by the formula:

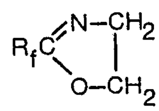

(III)

where $R_f$ is as defined above which comprises reacting a fluorine-containing ester represented by the formula:

$$R_fCOOR \qquad (I)$$

where $R_f$ is as defined above, and R is an alkyl group having from 1 to 10 carbon atoms, with a 2-bromoethylamine hydrobromide in the presence of a base.

Now, the present invention will be described in detail with reference to the preferred embodiments.

The reaction for the process of the present invention has been confirmed to proceed in the following two steps, e.g. by the isolation of the intermediate.

$R_fCO_2R$ + $BrCH_2CH_2NH_2.HBr$ (2-bromoethylamine hydrobromide) + $K_2CO_3$ (base)

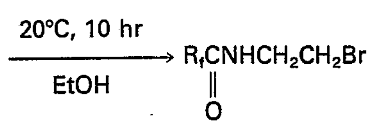


(hereinafter referred to as an intermediate) + ROH + KBr + $KHCO_3$ (IV)

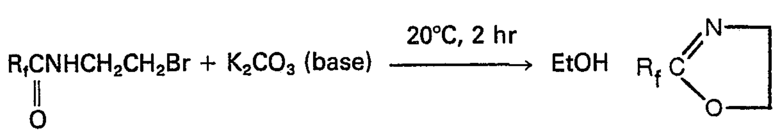


(2-(perfluoroalkyl)-1,3-oxazoline) + KBr + $KHCO_2$ (V)

In the reaction IV of the first step, the intermediate can be obtained in better yield by firstly reacting $BrCH_2CH_2NH_2.HBr$ with a base, and then with $R_fCO_2R$ in the presence of a base. For the reactions IV and V, it is important to select the optimum combination from the various bases and various reaction solvents. For instance, in the reaction IV, if $Et_2O$ (ethyl ether) is used as the reaction solvent, it is preferred to use $K_2CO_3$ as the base rather than $KHCO_3$ since it is thereby possible to obtain the intermediate in better yield. Likewise, in the reaction V, if $K_2CO_3$ is used as the base, 2-(perfluoroalkyl)-1,3-oxazoline is obtainable in better yield by using EtOH (ethanol) as the reaction solvent rather than MeOH (methanol).

In the present invention, as the base, there may be employed MeONa (sodium methylate), EtONa (sodium ethylate), $K_2CO_3$, $Na_2CO_3$, $Et_3N$ (triethylamine), NaOH, t-BuOK (potassium t-butylate). On the other hand, as the reaction solvent, it is preferred to use EtOH, $Et_2O$, THF (tetrahydrofuran) or $CH_3CN$. The reactions IV and V are preferably conducted at a normal temperature under atmospheric pressure. The reaction can be conducted at a temperature of from about −30 to 150°C. The reaction time varies between the reactions IV and V or depending upon the combination of the reaction solvent and the base used. However, both reactions are usually completed from a few minutes to a few hours. With respect to the amounts of the base and the reaction solvent in the reactions IV and V, the optimum amounts may be selected depending upon the types thereof or the types of the reactants. The base is used usually in an amount of from 0.3 to 10 times, preferably from 0.5 to 1.5 times by parts by weight relative to the starting material $R_fCO_2R$ or the intermediate. Likewise, the reaction solvent is used usually in an amount of from 2 to 20 times, preferably from 4 to 8 times.

2-(Perfluoroalkyl)-1,3-oxazolines obtained by the process of the present invention are highly reactive compounds having a perfluoroalkyl group, and useful as a starting material for highly durable water and oil repelling agents or as a starting material for surface active agents, or as a perfluoroalkyl group introducing agent for the preparation of medical or agricultural chemicals or as an amino ethylating agent for e.g. cellulose.

Now, the present invention will be described in further detail with reference to Examples.

Example 1

0.01 Mole of $CF_3\underset{\underset{\displaystyle O}{\|}}{C}OEt$

and 0.01 mol of $BrCH_2CH_2NH_2.HBr$ were reacted at a temperature of 20°C in the presence of a combination of 0.01 mol of a base and 15 ml of a reaction solvent to obtain an intermediate of

$$CF_3\underset{\underset{\displaystyle O}{\|}}{C}NHCH_2CH_2Br.$$

The results are shown in Table 1.

TABLE 1

| Base | Solvent | Reaction time (hr) | Reaction method* | Yield (%) |
|---|---|---|---|---|
| EtONa | EtOH | 1.0 | A | 83 |
| " | " | 1.0 | B-10 | 93 |
| " | " | 3.0 | B-10 | 92 |
| " | " | 3.0 | B-150 | 71 |
| $K_2CO_3$ | " | 10.0 | B-10 | 74 |
| " | $Et_2O$ | 10.0 | B-10 | 78 |
| " | THF | 10.0 | B-10 | 73 |
| " | $CH_3CN$ | 8.0 | B-10 | 59 |
| $Na_2CO_3$ | $Et_2O$ | 30.0 | B-10 | 70 |
| $Et_3N$ | " | 20.0 | B-10 | 89 |
| NaOH | " | 35.0 | B-30 | 64 |
| " | EtOH | 7.0 | B-10 | 81 |

* Reaction method
A: A base was added to a reaction solution of $CF_3COOEt$ with $BrCH_2CH_2NH_2.HBr$.
B-α: To the reaction solution of $BrCH_2CH_2NH_2.HBr$ with a base, $CF_3COOEt$ was added α minutes later.

Example 2

In 10 ml of a reaction solvent,

5 mmol of 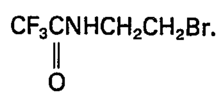

prepared in Example 1 and 5.5 mmol of a base, were reacted at a temperature of 20°C to obtain 2-(perfluoroalkyl)-1,3-oxazoline

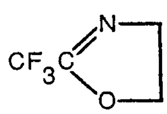 .

The results obtained by various combinations of the bases and reaction solvents are shown in Table 2.

TABLE 2

| Base | Solvent | Reaction time (hr) | Yield (%) |
|---|---|---|---|
| $K_2CO_3$ | EtOH | 2.0 | 88 |
| t-BuOK | $Et_2O$ | 1.0 | 92 |
| EtONa | EtOH | 0.25 | 65 |
| ″ | ″ | 1.5 | 53 |
| ″ | ′ | 4.0 | 50 |
| MeONa | $Et_2O$ | 0.25 | 93 |
| ″ | ″ | 0.5 | 84 |
| ″ | ″ | 1.0 | 81 |
| ″ | ″ | 2.0 | 80 |

Example 3

To 15 ml of EtOH, 0.01 mol of $BrCH_2CH_2NH_2.HBr$ and 0.021 mol of $K_2CO_3$ were added, and the mixture was stirred for 10 minutes. Then,

0.01 mol of $CF_3COEt$
‖
O

was added, and the mixture was reacted at 20°C for 7 hours, whereby 2-trifluoromethyl-1,3-oxazoline was obtained in a yield of 65%.

Example 4

0.01 Mol of $BrCH_2CH_2NH_2.HBr$ was dissolved in 10 ml of EtOH. To this solution, a EtONa/EtOH solution prepared by dissolving 0.01 mol of sodium metal in 5 ml of EtOH, was added. Ten minutes later,

0.01 mol of $CF_3COEt$
‖
O

was added, and the mixture was stirred for 3 hours. To this solution, 0.011 mol of $K_2CO_3$ was added, and the mixture was stirred for further 22 hours, whereby 2-trifluoromethyl-1,3-oxazoline was obtained in a yield of 72%.

Example 5

0.01 Mol of $C_8F_{17}COEt$
‖
O

and 0.01 mol of $BrCH_2CH_2NH_2.HBr$ were reacted in 15 ml of ethanol at 20°C for 7 hours in the presence of 0.01 mol of $K_2CO_3$, whereby 2-perfluorooctyl-1,3-oxazoline was obtained in a yield of 50%.

**Claims**

1. A process for producing an N-(β-bromoethyl)amide of a fluorine-containing carboxylic acid represented by the formula:

$R_fCNHCH_2CH_2Br$ (II)
‖
O

where $R_f$ is a perfluoroalkyl group having from 1 to 20 carbon atoms, and/or a fluorine-containing 2-oxazoline represented by the formula:

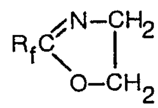

(III)

where $R_f$ is as defined above which comprises reacting a fluorine-containing ester represented by the formula:

$$R_fCOOR \quad (I)$$

where $R_f$ is as defined above, and R is an alkyl group having from 1 to 10 carbon atoms, with a 2-bromoethylamine hydrobromide in the presence of a base.

2. The process according to Claim 1, wherein the 2-bromoethylamine hydrobromide is reacted with a base and then with the compound of the formula I in the presence of a base.

## Patentansprüche

1. Verfahren zur Herstellung eines N-(β-Bromethyl)amids einer fluorhaltigen Carbonsäure der folgenden Formel

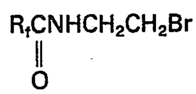

(II)

wobei $R_f$ für eine Perfluoralkylgruppe mit 1 bis 20 Kohlenstoffatomen steht, und/oder eines fluorhaltigen 2-Oxalins der folgenden Formel

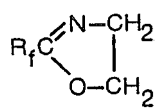

(III)

wobei $R_f$ wie oben definiert ist, wobei man einen fluorhaltigen Ester der Formel

$$R_fCOOR \quad (I)$$

wobei $R_f$ wie oben definiert ist und R eine Alkylgruppe mit 1 bis 10 Kohlenstoffatomen ist, mit einem 2-Bromethylaminhydrobromid in Gegenwart einer Base umsetzt.

2. Verfahren gemäß Anspruch 1, wobei das 2-Bromethylaminhydrobromid mit einer Base umgesetzt wird und anschließend mit einer Verbindung der Formel (I) in Gegenwart einer Base.

## Revendications

1. Procédé pour la préparation du N-(β-bromoéthyl)amide d'un acide carboxylique contenant du fluor, représenté par la formule:

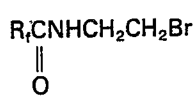

(II)

où $R_f$ représente un groupe perfluoroalkyle ayant de 1 à 20 atomes de carbone, et/ou d'une oxazoline contenant du fluor en position 2, représentée par la formule:

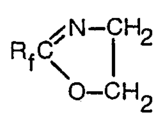

(III)

ou $R_f$ est tel que défini ci-dessus, qui consiste à faire réagir un ester contenant du fluor, représenté par la formule:

$$R_fCOOR \quad (I)$$

où:

— $R_f$ est tel que défini ci-dessus; et

— R représente un groupe alkyle ayant de 1 à 10 atomes de carbone,

avec le bromhydrate de la bromo-2 éthylamine, en présence d'une base.

2. Procédé selon la revendication 1, suivant lequel on fait réagir le bromhydrate de la bromo-2 éthylamine avec une base et, ensuite, avec le composé de formule I, en présence d'une base.